Europäisches Patentamt

European Patent Office

Office européen des brevets

(19)

(11) Publication number: **0 175 268**
A2

# EUROPEAN PATENT APPLICATION

(21) Application number: 85111460.3

(22) Date of filing: 11.09.85

(51) Int. Cl.⁴: **C 12 P 1/00,** A 61 K 35/80, G 01 N 33/50 // (C12P1/00, C12R1:89)

---

(30) Priority: **17.09.84 JP 194425/84**

(43) Date of publication of application: **26.03.86 Bulletin 86/13**

(84) Designated Contracting States: **AT BE CH DE FR GB LI NL SE**

(71) Applicant: **CHLORELLA INDUSTRY CO., LTD, 5th Floor, Toyokuni Building No. 4-6 Shiba Daimon 2-chome, Minato-ku Tokyo (JP)**

(72) Inventor: **Tanaka, Kuniaki, 1575-1, Arakimachiaraki, Kurume-shi Fukuoka-ken (JP)**
Inventor: **Konishi, Fumiko, 1770-14, Arakimachishirakuchi, Kurume-shiFukuoka-ken (JP)**

(74) Representative: **Strasse, Joachim, Dipl.-Ing. et al, Strasse und Stoffregen European Patent Attorneys Zweibrückenstrasse 17, D-8000 München 2 (DE)**

---

(54) Potentiator conferring resistance to infection.

(57) A potentiator conferring resistance to infection includes a substance having a molecular weight of 5,000 or more. The substance is contained in an extract extracted from chlorella alga using an aqueous solvent. The potentiator confers resistance to bacterial or viral infection on a person.

- 1 -

Potentiator conferring resistance to infection

The present invention relates to a potentiator conferring resistance to infection.

An animal has an host-defense mechanism against invasion of foreign substances to protect itself. The host-defense mechanism is classified into non-specific protection in which neutrophils and macrophages function, and specific immunity in which antibodies and sensitized lymphocytes function. Invading extracellular proliferative parasitic bacteria such as Escherichia coli and Pscudomonas aeruginosa are almost entirely excluded by non-specific protection. On the other hand, an invading tubercule bacillus or salmonella is killed by specific immunity. Cells having the host-defense mechanism destroy invading bacteria or viruses by producing various factors, e.g., lymphokine, active oxigen, chemotactic factor and interferon, causing cell interactions and activation of cells. Antibiotics and chemotherapentics are conventionally and frequently used as agents for external administration. However, a chlorella extract conferring resistance to infection has not been considered for this purpose.

It is an object of the present invention to provide an inexpensive potentiator for conferring resistance to bacterial or viral infections.

In order to achieve the above object of the present invention, there is provided a potentiator conferring resistance to infection, comprising a substance contained in an extract extracted from an alga belonging to genus Chlorella and having a molecular weight of 5,000 or more as an effective component.

A potentiator conferring resistance to infection according to the present invention is an agent having as an effective agent a substance of a molecular weight of 5,000 or more which is contained in a chlorella extract.

This substance can be prepared in the following manner. First, chlorella as a raw material is prepared. Any chlorella of the genus Chlorella can be used (e.g., C. vulgaris, C. regularis, C. eripsoidia, etc.). The culture method of an alga of the genus Chlorella is well known. More specifically, a chlorella alga can be cultured by a method requiring sunlight, carbon dioxide gas and various nutrients, i.e., heteroculture. For example, a chlorella alga can be cultured by agitation in an outdoor pool containing acetic acid or glucose as a carbon source, urea or the like as a nitrogen source, and potassium phosphate as a K and P source.

The cultured chlorella alga is subjected to extraction by an aqueous solvent. Before extraction, the alga is preferably spray dried. That is, the alga is sprayed in a hot air flow at 120°C or higher and dehydrated. Then, the alga is subjected to extraction by an aqueous solvent. The aqueous solvent used contains water, an aqueous solution containing an acid (e.g., 0.2 N hydrochloric acid) or a base (e.g., 0.2 N sodium hydroxide) having a pH of 5 to 9, and water-containing lower alcohols (e.g., methanol, ethanol). Although the spray-dried alga can be subjected to extraction by the aqueous solvent at 4 to 98°C for 15 minutes to 1 hour, it is preferably subjected to extraction by the aqueous solvent at 93 to 98°C for 15 to 40 minutes. The ratio of the alga and aqueous solvent during extraction can be selected to be, for example, 1 g of alga and 20 mℓ of aqueous solvent. In order to obtain an objective chlorella extract (to be referred to as CE hereinafter), the solvent extract is centrifuged to recover the CE as supernatant.

The thus obtained CE can be directly used as a potentiator conferring resistance to infection. However, in order to improve the effect as a potentiator, a fraction having a molecular weight of 5,000 or more (to be referred to as CVE-A hereinafter) is preferably recovered from the CE by purification. CVE-A having a molecular weight of 5,000 or more can be obtained by gel filtration chromatography or by dialysis using water. When gel filtration chromatography is performed, well known Sephadex G-25 (trade name) can be used. CVE-A is obtained through this purification process. CVE-A seems to contain various kinds of polysaccharides and proteins.

The CE or CVE-A obtained in this manner is used for a potentiator conferring resistance to infection. Before testing, the CE or CVE-A need not be subjected to any treatment. However, the CE or CVE-A as obtained may be lyophilized for purpose of easy handling.

The thus obtained CE or CVE-A is then administered to a subject. The administration can be performed orally, subcutaneously or intravenously. When a potentiator of the present invention is administered as CVE-A to an adult, the administration amount is 500 to 2,000 mg/day when oral administration is performed, and 50 to 200 mg/day when subcutaneous or intravenous administration is performed. When CVE-A is orally administered, it is preferably administered in pill or powder form. The potentiator can have a composition of 50 g of lyophilized CVE-A, 30 g of lactose and 20 g of dextran per 100 g. When CVE-A is administered subcutaneously or intravenously, a solution of lyophilized CVE-A must be prepared. In this case, the lyophilized CVE-A can be dissolved in a physiological saline solution in a concentration of 20 mg/m$\ell$, and 5 m$\ell$ of the resultant solution can be filled in an ampule. The $LD_{50}$ value of CVE-A is more than 4 g/kg when orally administered to a mouse and more than 400 mg/kg when subcutaneously or intravenously administered.

Example 1

Preparation of Testing Agent for
Potentiator Conferring Resistance to Infection

Chlorella vulgaris was cultured by conventional heteroculture. The culture was performed under agitation in an outdoor pool using acetic acid or glucose as a carbon source, urea or the like as a nitrogen source, and potassium phosphate as a K and P source. The collected chlorella alga was spray-dried in a hot air flow at 120°C or more. One gram of the dried chlorella alga was placed in 20 m$\ell$ of water at 93°C and was heated within a temperature range of 93 to 98°C for 15 minutes under frequent agitation. Thereafter, the water containing the alga was cooled to 4°C, and centrifuged at 6,000 rpm for 15 minutes. The supernatant was recovered to obtain CE. The CE was gel-filtrated with Sephadex G-25 (trade name) to obtain a fraction (CVE-A) having a molecular weight of 5,000 or more.

Example 2

Administration Test of CVE-A

CDF1 mice of the first generation of mixed breed BALB/c and DBA/2 inbred strain mice were each administered 1.0 mg of CVE-A intravenously, subcutaneously or intraperitoneally. One day after administration of CVE-A, each mouse was inoculated with enterotoxigenic E. coli by way of injection into the abdominal cavity. The doses of the E. coli were classified into three groups including the lethal dose as shown in Table 1. Inoculation was performed by

filling 1 mℓ of a suspension of enterotoxigenic E. coli in a phosphate buffed saline into an injector and injecting 0.2 mℓ of the suspension. The number of mice dying as a result was counted 5 days after the inoculation. As a control, the number of mice dying after infection of enterotoxigenic E. coli but not administered with CVE-A was similarly counted. The results are shown in Table 1. In Table 1, Table 2 of Example 3 and Table 3 of Example 4 to be described below, the inoculation amount represents the number of infecting bacteria per mouse and the mortality rate is represented by the number of dead mice/total number of mice tested.

Table 1

| No. of E. Coli Inoculation | Mortality Rate | | | |
|---|---|---|---|---|
| | Control | Intra-venous | Abdominal Cavity | Subcutane-ous |
| $2 \times 10^7$ | 4/6 | 0/6 | 0/6 | 0/6 |
| $8 \times 10^7$ | 6/6 | 0/6 | 0/6 | 1/6 |
| $3 \times 10^8$ | 6/6 | 6/6 | 6/6 | 6/6 |

As can be seen from Table 1, 4 or all mice in the control group died when inoculated with low or intermediate levels of enterotoxigenic E. coli. However, of groups of mice administered with CVE-A intravenously, intraperitoneally and subcutaneously none or only one died. This reveals that administration of CVE-A significantly confers resistance to bacterial infection.

Example 3

Administration of CVE-A

Mice were intraperitoneally administered CVE-A once following the same procedures as in Example 2, and each was inoculated with enterotoxigenic E. coli 1, 2, 4 and 7 days after the CVE-A administration following the same procedures as in Example 2. The mortality rate within 5 days after the inoculation was examined. As a control, the mortality rate of a control group of mice inoculated with enterotoxigenic E. coli but not administered CVE-A was similarly observed. The results are shown in Table 2. The number of days in the table is the number of days between administration of CVE-A and inoculation of enterotoxigenic E. coli.

Table 2

| No. of E. Coli Inoculation | Mortality Rate | | | | |
|---|---|---|---|---|---|
| | Control | 1 Day After Inocu-lation | 2 Days After Inocu-lation | 4 Days After Inocu-lation | 7 Days After Inocu-lation |
| $9 \times 10^6$ | 4/10 | 0/10 | 0/10 | 0/10 | 2/10 |
| $1 \times 10^8$ | 10/10 | 4/10 | 4/10 | 5/10 | 7/10 |

As can be seen from Table 2, as compared to the control group, the mortality rate is significantly lower for the group in which mice were inoculated with a low level of enterotoxigenic E. coli and administered CVE-A. This reveals that resistance to infection conferred by CVE-A can be maintained for a long period of time.

Example 4

Administration of CVE-A

The inventors examined improvements in the survival rates by administering CVE-A in mice which were treated with Cyclophosphamide and infected with E. coli. Cycolophosphamide (CY) which is an alkylating agent having carcinostatic effects decreases resistance to bacterial infection. The experiment was conducted as follows. CY was intraperitoneally administered to mice at a dose of 150 mg per kg of the mouse's weight. Three days after administration of CY, CVE-A was subcutaneously administered to the mice at doses listed in Table 4. One day after administration of CVE-A, enterotoxigenic E. coli was inoculated by way of injection into the abdominal cavity. The number of mice dying as a result was counted 5 days after inoculation. The results are shown in Table 3.

Table 3

| Treatment with | | | Survival | |
|---|---|---|---|---|
| CY | CVE-A | E. coli | No. of E. coli Inoculation | |
| on day | | | | |
| -4 | -1 | 0 | $4 \times 10^6$ | $2 \times 10^7$ |
| − | − | + | 9/10 | 4/10 |
| + | − | + | 4/10 | 0/10 |
| + | 0.5 mg/kg | + | 8/10 | 0/10 |
| + | 5.0 mg/kg | + | 9/10 | 2/10 |
| + | 50 mg/kg | + | 8/10 | 7/10 |

As can be seen from Table 3, administration of CY causes a decrease in the survival rates. However, additional administration of CVE-A improves the survival rates.

Such an effect was similarly obtained when mice were inoculated with Meth A fibrosarcoma as one type of cancer specifically occurring in mice and were infected with E. coli. In general, a major factor causing death in patients with cancer is considered to be a decrease in resistance of a patient to bacterial or viral infection.

When a potentiator of the present invention is administered to a patient having a impaired resistance to infection, including cancer patients, the patient can be protected from bacteria or viruses, thus providing an important effect.

Claims:

1.  A potentiator conferring resistance to infection comprising as an effective component a substance having a molecular weight not less than 5,000 contained in an extract extracted from a chlorella alga by an aqueous solvent.

2.  A testing agent according to claim 1, characterized in that the substance is extracted in the aqueous solvent at 4 to 98°C for 15 minutes to 30 hours.

3.  A testing agent according to claim 2, characterized in that the aqueous solvent is water and extraction is performed at 93 to 98°C for 15 to 40 minutes.

4.  A potentiator conferring resistance to infection comprising as an effective component a substance having a molecular weight of not less than 5,000 which is obtained by extraction of a chlorella alga by an aqueous solvent and purifying the extract by gel filtration chromatography or by dialysis against water.